# EUROPEAN PATENT APPLICATION

(11) **EP 1 783 138 A1**
(43) Date of publication of application: **09.05.2007**
(21) Application number: 05767198.4
(22) Date of filing: 22.07.2005
(51) Int. Cl.: C07K 1/20, C07K 14/115, C12N 7/02

(54) **METHOD OF PURIFYING VIRUS ENVELOPE**

(30) Priority: 27.07.2004 JP 2004219381
(71) Applicant: GenomIdea Inc., Osaka 567-0085 (JP)
(72) Inventor: IOKA, Shinichi, 6340845 (JP)
(74) Representative: Wichmann, Hendrik
(86) International application number: PCT/JP2005/013893
(87) International publication number: WO 2006/011580

(57) **Abstract**

An industrial purification method of a virus (e.g., Hemagglutinating Virus of Japan, HVJ) envelope is provided. To be specific, a method of purifying an inactivated virus envelope at a high recovery rate by ion exchange chromatography and hydrophobic chromatography, while maintaining the cell fusion activity of the virus, is provided. The purified virus envelope can be used as a vector for introducing a biopolymer such as gene and the like into a cell or a living organism. In addition, this method can be used for purification of an attenuated envelope virus.

## Description

### Technical Field

The present invention relates to an industrial purification method of a virus (e.g., Hemagglutinating Virus of Japan, hereinafter referred to as HVJ) envelope. A purified virus envelope can be used as a vector for introducing a biopolymer such as a gene and the like into a cell and a living organism.

### Background Art

Many viral methods and non-viral methods have been developed for the purpose of introducing a gene into cultured cells and living tissues for functional analyses of gene and gene therapy (Mulligan, Science, 260, 926-932, 1993 and Ledley, Human Gene Therapy, vol.6, 1129-1144, 1995). For introduction of a gene into a cell, a viral method is generally effective. However, the method using a viral vector has a safety problem because of the possibility of parental virus-derived gene introduction and its expression, immunogenicity, and the possibility of modification of host genome structure. On the other hand, many of the non-viral methods using a liposome and the like show lower cytotoxicity and lower immunogenicity than do the viral methods. However, the efficiency of the gene introduction into cultured cells and living tissues tends to be lower than with viral vectors.

HVJ is a virus belonging to the genus *Paramyxoviridae,* which has an envelope, and hemagglutinin and neuraminidase on the surface of the envelope. HVJ attracted attention for fusing Ehrlich tumor cells (Okada, Biken Journal, 1, 103-110, 1958), and analysis of cellular membrane fusion activity (hereinafter fusion activity) has been undertaken and its use as a transgenic vector has been studied. HVJ has high immunogenicity and is known to induce CTL particularly when NP protein is produced in a large amount (Cole G.A. et al. Journal of Immunology 158, 4301-4309, 1997). Moreover, inhibition of synthesis of protein by a host is feared. Thus, a method of preparing fused particles (HVJ-liposome) by fusing a liposome including a gene or a protein with HVJ inactivated by ultraviolet irradiation in advance was devised, by which noninvasive gene introduction into a cell or a living organism has been enabled (US Patent No. 5,631,237, Dzau et al., Proc. Natl. Acad. Sci. USA, 93, 11421-11425, 1996 and Kaneda et al., Molecular Medicine Today, 5, 298-303, 1999). However, this method is complicated in that two different vesicles of a virus and a liposome need to be prepared. In addition, it has been clarified that HVJ-liposome has an average diameter of 1.3 times larger than that of HVJ particles and shows a fusion activity decreased to not more than one-tenth of HVJ alone.

Kaneda developed an HVJ vector which introduces a gene into a cell at high efficiency and shows high safety (WO01/57204). To be specific, the genome of an envelope virus including HVJ is inactivated, and a biopolymer such as a gene and the like is included in its envelope, and the virus is used as a vector to be introduced into a cell or living organism. At this stage, however, an efficient purification method of an HVJ envelope has not been established.

Furthermore, Kaneda developed a method including producing HVJ in a hen egg, and purifying the HVJ by the steps of filtration, membrane concentration, ultrafiltration, ion exchange chromatography, and ultrafiltration (WO03/014338). Since this method includes two treatment steps of filtration and membrane concentration before column, however, the physical stimuli degrade the activity of HVJ, and degradation of recovery rate due to capture at this stage is inevitable.

As a purification method of virus, a purification method of adenovirus, which uses an ion exchange resin and an immobilized metal affinity resin, has been developed in WO96/27677. Since adenovirus does not have an envelope, however, this method is not applicable as a purification method of an envelope virus. While a method using an ion exchange resin and a hydrophobic resin in combination is proposed as a purification method of adenovirus, a concrete example is not disclosed.

While WO91/00104 discloses a method of purifying the HN protein and the F protein of an envelope virus by affinity chromatography, the method is not applicable to a method of purifying a virus envelope, which is an aggregate of many proteins.

Thus, the development of a purification method to efficiently produce a virus envelope, which solves the above-mentioned problems, and shows a higher recovery rate while maintaining the activity of HVJ, has been desired.

### Disclosure of the Invention

The challenge of the present invention is to develop a method of purifying a virus envelope at a higher recovery rate while maintaining the fusion activity of the virus.

Envelope viruses including HVJ are conjugated proteins consisting of several kinds of proteins, lipids and sugar chains, and are generally large particles with a particle size of several hundred nanometers. Therefore, they were expected to have a complicated surface charge and very high hydrophobicity. Thus, recent chromatography carriers allegedly having high separation ability, which have a smaller pore size (bore of one of many pores on the carrier surface) and a small surface area per unit, and designed to separate comparatively small molecules, are incapable of sufficiently utilizing various interactions between HVJ and the carrier surface. In other words, to separate a large and highly hydrophobic molecule such as HVJ, a loose pore size or sufficient surface area permitting distribution of HVJ inside a carrier, a sufficient distance between functional groups permitting binding of HVJ, and an interaction with a sufficient binding constant and an appropriate dissociation constant are necessary.

A culture supernatant contains various impurities other than HVJ. However, most of them are molecules or particles smaller than HVJ. Since most of the chromatography carriers of the present day are made to have selectivity for the small-sized molecule side, they cannot separate HVJ appropriately from a culture supernatant. Therefore, there is a need to consider a purification method suitable for HVJ. The present inventor has conducted intensive studies in an attempt to solve the above-mentioned problems. As a result, he has established a purification method comprising hydrophobic chromatography, preferably hydrophobic chromatography and anion exchange chromatography and/or gel filtration chromatography in combination.

That is, the present invention provides, as a means of solving the problems, a method for efficiently purifying a virus envelope with high recovery rate, while maintaining the cell fusion activity of the virus envelope. The subject matter thereof relates to
(1) a method of purifying a virus envelope, comprising use of hydrophobic chromatography,
(2) the method of (1), wherein a functional group of the aforementioned hydrophobic chromatography is a weak hydrophobic group,
(3) the method of (1) or (2), wherein a functional group of the aforementioned hydrophobic chromatography is an oligoethylene glycol group or a phenyl group,
(4) the method of (1) to (3), wherein a functional group of the aforementioned hydrophobic chromatography is an oligoethylene glycol group,
(5) the method of (1) to (4), wherein the aforementioned hydrophobic chromatography and ion exchange resin chromatography and/or gel filtration chromatography are combined,
(6) the method of (5), wherein the ion exchange chromatography and hydrophobic chromatography are combined,
(7) the method of (5) or (6), wherein an ion exchange resin is used for the first chromatography and a hydrophobic resin is used for the second chromatography,
(8) the method of (5) to (7), wherein the ion exchange chromatography is anion exchange chromatography,
(9) the method of (5) to (8), wherein the ion exchange chromatography is weak anion exchange chromatography,
(10) the method of (5) to (9), wherein a functional group of the ion exchange chromatography is a diethylaminopropyl (DEAP) group,
(11) the method of (5) to (10), wherein a functional group of the ion exchange chromatography is a low density substituted (Low Sub),
(12) the method of (1) to (11), wherein a buffer for the chromatography has pH 7-9,
(13) the method of (1) to (12), wherein a buffer for the chromatography has pH 7.8-8.5,
(14) the method of (5) to (13), wherein a buffer for the ion exchange chromatography comprises sodium chloride or potassium chloride,
(15) the method of (5) to (14), wherein a buffer for sample adsorption in the ion exchange chromatography comprises 0.01-150 mM of sodium chloride,
(16) the method of (5) to (15), wherein a buffer for sample adsorption in the ion exchange chromatography comprises 30-70 mM of sodium chloride,
(17) the method of (5) to (16), wherein a buffer for washing in the ion exchange chromatography comprises 150 mM-250 mM of sodium chloride,
(18) the method of (5) to (17), wherein a buffer for washing in the ion exchange chromatography comprises 190-230 mM of sodium chloride,
(19) the method of (5) to (18), wherein a buffer for elution in the ion exchange chromatography comprises 100 mM-1000 mM of sodium chloride,
(20) the method of (5) to (19), wherein a buffer for elution in the ion exchange chromatography comprises 290-330 mM of sodium chloride,
(21) the method of (1) to (20), wherein a buffer for the hydrophobic chromatography comprises ammonium sulfate, sodium sulfate or sodium chloride,
(22) the method of (1) to (21), wherein a buffer for adsorption in the hydrophobic chromatography comprises 1.0 M-2.5 M of ammonium sulfate,
(23) the method of (1) to (22), wherein a buffer for adsorption in the hydrophobic chromatography comprises 1.8-2.2 M of ammonium sulfate,
(24) the method of (1) to (23), wherein a buffer for washing in the hydrophobic chromatography comprises 1.0-1.6 M of ammonium sulfate,
(25) the method of (1) to (24), wherein a buffer for washing in the hydrophobic chromatography comprises 1.2-1.5 M of ammonium sulfate,
(26) the method of (1) to (25), wherein a buffer for elution in the hydrophobic chromatography comprises 0.01-1.5 M of ammonium sulfate,
(27) the method of (1) to (26), wherein a buffer for elution in the hydrophobic chromatography comprises 0.6-1.0 M of ammonium sulfate,
(28) the method of (1) to (27), wherein a buffer for elution in the hydrophobic chromatography comprises a hydrophilic organic solvent,
(29) the method of (28), wherein the hydrophilic organic solvent is polyvalent alcohol or lower alcohol,
(30) the method of (28) or (29), wherein the polyvalent alcohol is ethylene glycol,
(31) the method of (28) to (30), wherein a concentration of ethylene glycol is 0.01-50%,
(32) the method of (28) to (31), wherein the concentration of ethylene glycol is 2-10%,
(33) the method of (28) to (32), wherein the concentration of ethylene glycol is 3-7%,
(34) the method of (1) to (33), wherein a buffer for elution in the hydrophobic chromatography comprises a surfactant,
(35) the method of (1) to (34), wherein the aforementioned surfactant is Tween 80 or Triton X,
(36) the method of (1) to (35), wherein a buffer for elution in the hydrophobic chromatography comprises 0.001-1% of Tween 80,
(37) the method of (1) to (36), wherein a buffer for elution in the hydrophobic chromatography comprises 0.01-0.1% of Tween 80,
(38) the method of (1) to (37), wherein, in the hydrophobic chromatography, elution after sample adsorption is caused by lowering the temperature by not less than 5°C from the temperature during adsorption,
(39) the method of (38), wherein the temperature during elution is within the range of (room temperature - 5)°C - 4°C,
(40) the method of (1) to (39), wherein, in the hydrophobic chromatography, elution after sample adsorption is caused by raising the pH of the buffer for adsorption by not less than 0.5,
(41) the method of (40), wherein the pH during elution is within the range of 6-10,
(42) the method of (1) to (41), wherein gel filtration chromatography is conducted after the hydrophobic chromatography,
(43) the method of (1) to (42), wherein a carrier of the gel filtration chromatography is Sepharose,
(44) the method of (1) to (43), which comprises adding a divalent metal ion to a buffer used for the gel filtration chromatography,
(45) the method of (44), wherein the divalent metal ion is calcium or magnesium,
(46) the method of (44) or (45), wherein a concentration of the divalent metal ion is 0.1-10 mM,
(47) the method of (44) to (46), wherein a concentration of the divalent metal ion is 1-2 mM,
(48) the method of (1) to (47), wherein the virus belongs to a family selected from the group consisting of *Filoviridae, Bunyaviridae, Herpesviridae, Poxviridae, Togaviridae, Coronaviridae, Flaviviridae, Paramyxoviridae, Arenaviridae, Orthomyxoviridae, Retroviridae, Hepadnaviridae, Reoviridae* and *Deltaviridae,*
(49) the method of (1) to (48), wherein the virus is selected from the group consisting of Ebola hemorrhagic fever virus, Crimean-Congo hemorrhagic fever virus, hantavirus, herpes simplex virus, EB virus, smallpox virus, cowpox virus, rubella virus, SARS virus (human coronavirus), hepatitis C virus, Japanese encephalitis virus, yellow fever virus, dengue fever virus, West Nile virus, Russian spring-summer encephalitis virus, hog cholera virus, rabies virus, vesicular stomatitis virus, Hemagglutinating Virus of Japan (HVJ), measles virus, epidemic parotiditis virus, mumps virus, rubella virus, RS virus, Lassa virus, influenza virus, human immunodeficiency virus (HIV), human T-cell leukemia virus type 1 (HTLV-1), feline immunodeficiency virus (FIV), hepatitis B virus (HBV), reovirus and hepatitis D virus,
(50) the method of (1) to (49), wherein the virus is HVJ,
(51) the method of (1) to (50), wherein the virus envelope is an attenuated or inactivated virus envelope,
(52) the method of (1) to (51), wherein the virus envelope is an inactivated virus envelope.

In addition, a virus envelope purified by the present invention can be utilized widely as a vector for introducing a low-molecule or high-molecule compound into a cell or a living organism. For example, as disclosed in WO2004/039406, a chemotherapeutic agent is included and the envelope can be used as an anticancer agent. As shown in WO2004/046353, moreover, any nucleic acid can be encapsulated and the envelope can be used for screening for the objective gene or protein.

### Brief Description of the Drawings

Fig. 1 shows an anion exchange chromatogram of column 1 (Example 1).
Fig. 2 shows a hydrophobic chromatogram of column 2 (Example 1).
Fig. 3 shows a gel filtration chromatogram of column 3 (Example 1).
Fig. 4 shows an SDS polyacrylamide electrophoretic image of a purified HVJ envelope (Example 3).
   Lane 1: molecular-weight marker, lane 2: cell-derived HVJ (reduced), lane 3: hen egg-derived HVJ (reduced), lane 4: cell-derived HVJ (non-reduced), lane 5: hen egg-derived HVJ (non-reduced).

### Best Mode for Embodying the Invention

When used in the present specification, the "gene introduction" means introducing a desired natural, synthetic or recombinant gene or gene segment into the target cell in a living organism or in vitro, in such a manner that the introduced gene can maintain its function. The gene or gene segment introduced in the present invention encompasses DNA, RNA or a nucleic acid, which is a synthetic analog thereof, having a particular sequence. When used in the present specification, moreover, gene transfer, transfection and transfect are used to show the same meaning (concept).

When used in the present specification, the "gene vector", "transgenic vector" and "virus envelope vector" mean a vector having an exogenous gene encapsulated in a virus envelope. The virus used for the preparation of a transgenic vector may be a wild-type virus or a recombinant virus.

In one aspect of the present invention, the virus to be used belongs to a family selected from the group consisting of *Filoviridae, Bunyaviridae, Herpesviridae, Poxviridae, Togaviridae, Coronaviridae, Flaviviridae, Paramyxoviridae, Arena viridae, Orthomyxovi ridae, Retroviridae, Hepadnaviridae, Reoviridae and Deltaviridae.*

Preferably, the virus is selected from the group consisting of Ebola hemorrhagic fever virus, Crimean-Congo hemorrhagic fever virus, hantavirus, herpes simplex virus, EB virus, smallpox virus, cowpox virus, rubella virus, SARS virus (human coronavirus), hepatitis C virus, Japanese encephalitis virus, yellow fever virus, dengue fever virus, West Nile virus, Russian spring-summer encephalitis virus, hog cholera virus, rabies virus, vesicular stomatitis virus, Hemagglutinating Virus of Japan (HVJ), measles virus, epidemic parotiditis virus, mumps virus, rubella virus, RS virus, Lassa virus, influenza virus, human immunodeficiency virus (HIV), human T-cell leukemia virus type 1 (HTLV-1), feline immunodeficiency virus (FIV), hepatitis B virus (HBV), reovirus and hepatitis.D virus.

More preferably, the virus is HVJ.

When used in the present specification, the "inactivated virus" means a virus having an inactivated genome. The inactivated virus is replication deficient. Preferably, the inactivation is performed by a UV treatment or a treatment with an alkylating agent.

When used in the present specification, the "attenuated virus" means a virus that does not show or hardly shows pathogenicity to a host even after infecting the host.

When used in the present specification, the "exogenous gene" means a nucleic acid sequence included in a transgenic vector, which is derived from an origin other than virus. In one aspect of the present invention, the exogenous gene is operably linked to a regulatory gene (e.g., promoter, enhancer, terminator and poly A additional signal, necessary for transcription, as well as liposome binding site, initiation codon, stop codon and the like, necessary for translation) suitable for expression of a gene introduced by a transgenic vector. In another aspect of the present invention, the exogenous gene does not contain a regulatory sequence for the expression of the exogenous gene. In a further aspect of the present invention, the exogenous gene is an oligonucleotide or decoy nucleic acid. While the exogenous gene included in a transgenic vector is represented by a nucleic acid molecule of DNA or RNA, the nucleic acid molecule to be introduced may contain a nucleic acid analog molecule. The molecular species included in a transgenic vector may be a single gene molecular species or plural, different gene molecular species.

In the present specification, "HVJ" and "Hemagglutinating Virus of Japan" are used to refer to the same meaning(concept).

In the present specification, the "HAU" refers to the viral activity capable of aggregating the chicken red cell 0.5%, where 1 HAU corresponds to almost 24 million virus particles (Okada, Y et al., Biken Journal 4, 209-213, 1961).

The purification method of the present invention can be performed with hydrophobic chromatography alone. Preferably, hydrophobic chromatography and ion exchange chromatography and/or gel filtration chromatography are used. Specifically, any of the following combinations is included.
(1) hydrophobic chromatography
(2) a combination of hydrophobic chromatography and ion exchange chromatography (in no particular order)
(3) a combination of hydrophobic chromatography and gel filtration chromatography (in no particular order)
(4) a combination of hydrophobic chromatography, ion exchange chromatography and gel filtration chromatography (in no particular order)

Most preferably, three step column chromatography is used. Since the third step aims at desalting and concentration, this step is applied as necessary. The pH of the buffer used for chromatography is generally 7-9. The pH is preferably 7.8-8.5.

Generally, anion exchange chromatography can be used for column 1. Theoretically, almost any anion exchanger (DEAP, Q, QAE, DEAE and the like) can be used (DEAP; diethyl aminopropyl, Q; quaternary amine, QAE; quaternary aminoethyl, DEAE; diethylaminoethyl). Preferable concrete examples include ANX-Sepharose 4FF with DEAP (GE Amersham Biosciences K.K., Cat. No. 17-1286-01). This exchanger is a carrier specialized in trapping high molecules since it has a comparatively large pore size, and the propyl group of DEAP group becomes a spacer and the distance between the carrier and the functional group is elongated. By achieving low density of the functional group of ANX-Sepharose4FF Low Sub, it has selectivity for large molecules rather than small molecules. This is because plural functional groups are generally bonded to one molecule of protein by the interaction of chromatography. Since general chromatography carriers are bound with a large excess of functional groups, an extremely high dense environment is generated. Since a carrier has a structure of entangled strings, it affords an environment advantageous to low molecules that can be spatially bonded to a greater number of points. In other words, ANX-Sepharose4FF has a steric space of functional group, that has been improved for high molecules. As a buffer to be used for ion exchange chromatography, a buffer having pH 6-9 can be used and, for example, Tris-HCl buffer, phosphate buffer, HEPES buffer and the like can be mentioned.

For column 2, hydrophobic chromatography can be generally used. Examples of preferable carrier to be used for hydrophobic chromatography include commercially available hydrophobic carriers with weak hydrophobicity and, for example, hydrophobic carriers having an ether group, an alkyl group and the like can be used. Particularly, a carrier having an ether group is preferable. Concrete examples of preferable carrier include Ether-TOYOPEARL650M (Cat. No. 16173) manufactured by TOSOH CORPORATION, which has an oligoethylene glycol group as its functional group. The oligoethylene glycol group is considered to show weak hydrophobicity because it has an OH group side chain in the carbon chain. A butyl group and a phenyl group of a carrier generally used for hydrophobic chromatography show too strong a binding force to a protein having high hydrophobicity. As a result, they prevent sufficient separation of the protein and decrease the recovery rate. This carrier was developed to prevent such decrease in the recovery rate. In addition, it is assumed that the hydrophobicity of TOYOPEARL is lower than that of the products of other companies, because the functional group is bonded by a method without a spacer. This is predictable from the comparison data of Phenyl-Sepharose (with a spacer) and Phenyl-TOYOPEARL. In general, purification of virus by hydrophobic chromatography having a butyl group, a phenyl group and the like is difficult. However, even smaller viruses can be purified when the functional group has a lower density than that of general products (commercially available products). As a buffer for hydrophobic chromatography, one having pH 6-9 can be used and, for example, Tris-HCl buffer, phosphate buffer, HEPES buffer, Bis-Tris/HCl, Bis-Tris propane/HCl, triethanolamine/HCl, triethanolamine/acetic acid, N-methyldiethanolamine/HCl, N-methyldiethanolamine/acetic acid, diethanolamine/HCl, 1,3-diaminopropane/HCl, piperazine/HCl, trimethylamine/HCl, ethanolamine/HCl, n-methylmorpholine/HCl and the like can be mentioned. HVJ bound to the oligoethylene glycol group can be eluted by lowering the concentration of ammonium sulfate in the buffer. With only such elution, however, the peak is not sharp and a considerable liquid amount is necessary to complete the elution. To increase the polarity of the solution, therefore, addition of a hydrophilic organic solvent is effective. The hydrophilic organic solvent can be added within the range of 0.01-50%. While the concentration is not limited, it is generally 2%-10%, preferably 3-7%, most preferably 5%. As the hydrophilic organic solvent, polyvalent alcohol or lower alcohol is preferably used. The lower alcohol in the present invention is not limited as long as it is C₁₋₆ lower hydrocarbon added with one hydroxyl group. To be specific, for example, methanol, ethanol, n-propanol, i-propanol, n-butanol, i-butanol, t-butanol and the like can be mentioned. In addition, polyvalent alcohol is not limited as long as it is C₂₋₆ hydrocarbon added with not less than two hydroxyl groups. To be specific, for example, ethylene glycol, propylene glycol, trimethylene glycol, glycerol and the like can be mentioned. More preferably, polyvalent alcohol is used. Most preferably, ethylene glycol is used. Ethylene glycol can be added within the range of 0.01-50%. While the concentration is not limited, it is generally 2%-10%, preferably 3-7%, most preferably 5%. Addition of ethylene glycol sharpens the peak, the elution volume becomes about the half of the column volume, and purification and concentration can be simultaneously performed. In this step, moreover, due to the purification at a high ammonium sulfate concentration, HVJ may be bonded to each other. To prevent such binding, it is effective to add a surfactant to an elution buffer. The surfactant can be added within the range of 0.001-1%, preferably 0.01-0.1%. As the surfactant, Tween and Triton X can be used. More preferably, Tween 80 is used. Tween 80 can be added within the range of 0.001-1%. Tween 80 is preferably added at 0.01-0.1%. Most preferably, it is added at 0.05%.

Furthermore, gel filtration chromatography can be generally used for column 3. Since this step is performed for concentration and desalting, widely used gel filtration chromatography can be employed. For example, Sepharose4FF (Cat. No. 17-0149-01) and Sepharose6FF (Cat. No. 17-0159-01) of GE Amersham Biosciences K.K. can be used. As a buffer to be used for gel filtration, a buffer having pH 6-9 can be used and, for example, Tris-HCl buffer, phosphate buffer, HEPES buffer, Bis-Tris/HCl, Bis-Tris propane/HCl, triethanolamine/HCl, triethanolamine/acetic acid, N-methyldiethanolamine/HCl, N-methyldiethanolamine/acetic acid, diethanolamine/HCl, 1,3-diaminopropane/HCl, piperazine/HCl, trimethylamine/HCl, ethanolamine /HCl, n-methylmorpholine/HCl and the like can be mentioned. The buffer may contain a hydrophilic organic solvent and, as the hydrophilic organic solvent that can be added, those similar to the above-mentioned can be used.

Moreover, addition of a divalent metal ion as a stabilizer of neuraminidase to a buffer to be used for gel filtration chromatography and/or final purified preparation is effective. The divalent metal ion is preferably calcium or magnesium. Generally, these metal ions are added within the range of 0.1-10 mM. Preferably, they are added to a concentration of 1-2 mM.

### Examples

The present invention is explained in detail in the following by referring to Examples, which are not to be construed as limitative.

### Example 1. purification of HVJ by column chromatography

HVJ produced in a floating cell culture system was subjected to centrifugation to perform a cell removal treatment. The obtained cell culture supernatant was purified by 3-stage chromatography.

First, for inactivation of endotoxin, a 0.25 M sodium hydroxide solution was passed through each column before use, and the column was used after standing for not less than 8 hr.

### (1) ion exchange resin chromatography

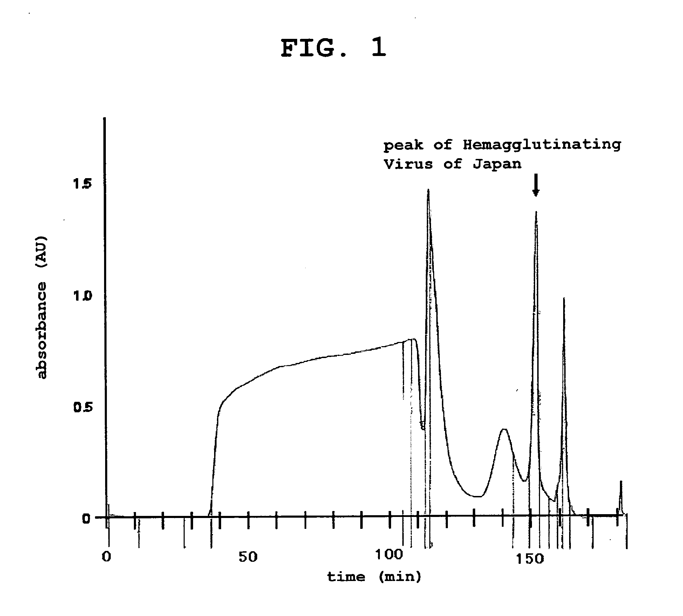

The centrifuged culture supernatant was diluted 2-fold with 50 mM Tris-HCl buffer (pH 7.8). This was passed through column 1 equilibrated with 50 mM Tris-HCl buffer (pH 7.8) containing 50 mM sodium chloride to allow adsorption. As column 1, ANX-Sepharose4FF Low Sub (manufactured by GE Amersham Biosciences K.K., Cat. No. 17-1286-01), which is a carrier for anion exchange chromatography, packed in a BPG200/500 empty column (manufactured by GE Amersham Biosciences K.K.) to diameter 20 cm, height 9 cm (3L) was used. The linear flow rate was 96 cm/h. Further, a 3-fold column volume of the equilibrating buffer was passed and then the column was washed by passing through a 8-fold column volume of 50 mM Tris-HCl buffer (pH 7.8) containing 210 mM sodium chloride. For elution of HVJ from column 1, 50 mM Tris-HCl buffer (pH 7.8) containing 310 mM sodium chloride was passed. At this time, since the absorbance of the column passing solution at UV 280 nm increased, the corresponding portion was recovered. This was used as a column 1 elution fraction. Finally, 50 mM Tris-HCl buffer containing 2 M sodium chloride was passed to regenerate column 1 (see, Fig. 1).

### (2) hydrophobic resin chromatography

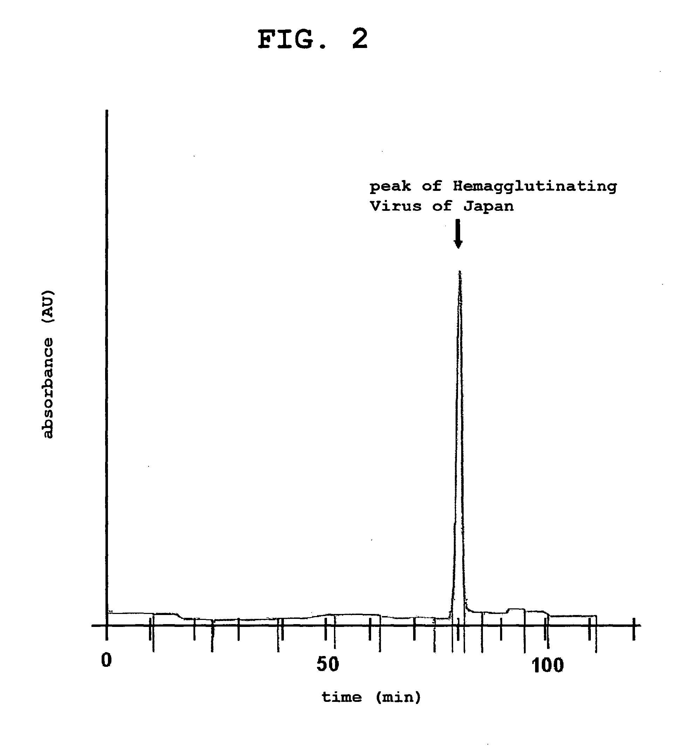

Then, an equal volume of 4M ammonium sulfate solution was added to column 1 eluted fraction to give a solution containing 2M ammonium sulfate. The mixture was passed through column 2 equilibrated with 50 mM Tris-HC1 buffer (pH 7.8) containing 2 M ammonium sulfate to allow adsorption. As column 2, Ether-TOYOPEARL 650M (manufactured by TOSOH, Cat. No. 16173), which is a carrier for hydrophobic interaction chromatography, packed in a BPG100/500 empty column (manufactured by GE Amersham Biosciences K.K.) to diameter 10 cm, height 12.5 cm (1 L), was used. The linear flow rate was 190 cm/h. Further, a 3-fold column volume of the equilibrating buffer was passed and then the column was washed by passing through a 5-fold column volume of 50 mM Tris-HCl buffer containing 1.4M ammonium sulfate. For elution of HVJ from column 2, 50 mM Tris-HCl buffer (pH 7.8) containing 0.8 M ammonium sulfate, 5% ethylene glycol, and 0.05% Tween 80 was passed. At this time, since the absorbance of the column passing solution at UV 280 nm increased, the corresponding portion was recovered. This was used as a column 2 eluted fraction. Finally, 5% ethylene glycol solution was passed to regenerate column 2. (see, Fig. 2)

### (3) gel filtration chromatography

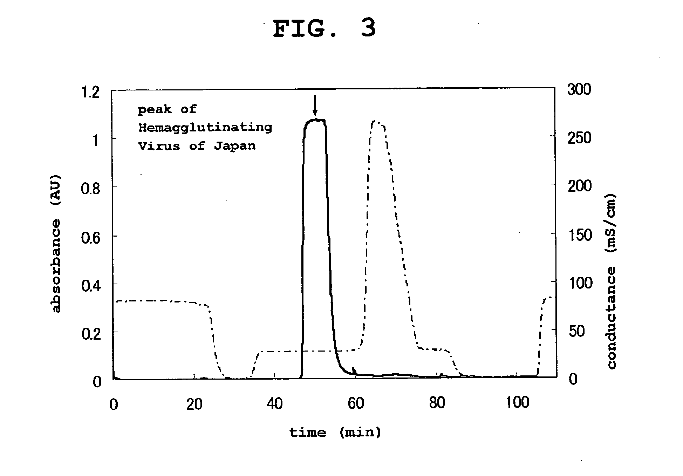

Furthermore, the column 2 eluted fraction was passed through column 3 equilibrated with 50 mM Tris-HCl buffer (pH 7.8) containing 150 mM sodium chloride. As column 3, Sepharose4FF (manufactured by GE Amersham Biosciences K.K., Cat. No. 17-0149-01), which is a carrier for gel filtration chromatography, packed in a XK50/60 empty column (manufactured by GE Amersham Biosciences K.K.) to diameter 5 cm, height 40 cm (0.8 L), was used. The linear flow rate was 120 cm/h. Following the column elution fraction, an equilibrating buffer was passed. At this time, since the absorbance of the column passing solution at UV 280 nm increased, the corresponding portion was recovered. This was used as a column 3 eluted fraction. (see, Fig. 3)

0.5% Methylcellulose in an amount corresponding to 1/4 volume was added to the column 3 eluted fraction to give a HVJ solution containing a final concentration of 0.1% methylcellulose. This was passed through a 0.45 micron sterile filtration filter. The mixture was quickly frozen with liquid nitrogen and preserved at - 80°C. As a result, highly pure HVJ could be stably preserved for a long time.

Of the main reagents used for buffer etc., tris(hydroxymethyl)aminomethane, sodium chloride, calcium chloride 2-hydrate, magnesium chloride 6-hydrate, sodium hydroxide, ethylene glycol, ammonium sulfate, and Tween 80 used were special grade products manufactured by Wako Pure Chemical Industries, Ltd. or Sigma Ltd. Hydrochloric acid used was 6N standard product manufactured by Wako Pure Chemical Industries, Ltd. 0.5% Methylcellulose added as a stabilizer for a purified preparation was one manufactured by Wako Pure Chemical Industries, Ltd. For sterilization by filtration of a purified preparation, hydrophilic filtration filter Millipak Gamma Gold (Nihon Millipore K.K.) having a pore diameter of 0.45 micron was used.

### Comparative Example 1. purification of HVJ by membrane concentration and column chromatography

HVJ produced in a floating cell culture system was subjected to centrifugation to perform a cell removal treatment. The obtained cell culture supernatant was purified by ultramembrane concentration and 3-stage chromatography.

First, for inactivation of endotoxin, a 0.25 M sodium hydroxide solution was passed through a membrane concentration apparatus and each column before use, and they were used after standing for not less than 8 hr (generally, standing for about one week completely eradicates viable cells and endotoxin).

### (1) filtration and membrane concentration

The centrifuged culture supernatant was passed through a 1.2 µm milli-grid filter to remove particles. The supernatant was concentrated to 600 mL using Pellicon membrane concentration apparatus.

### (2) gel filtration chromatography (column 1)

The concentrated culture supernatant obtained in (1) was passed through column 1 equilibrated with 50 mM Tris-HCl buffer (pH 7.8) containing 150 mM sodium chloride. As column 1, Sepharose6FF (manufactured by GE Amersham Biosciences K.K.), which is a carrier for gel filtration chromatography, packed in a BPG100/500 empty column (manufactured by GE Amersham Biosciences K.K.) to diameter 10 cm, height 33 cm (2.5 L), was used. The linear flow rate was 150 cm/h. Then an equilibrating buffer was passed. At this time, since the absorbance of the column passing solution at UV 280 nm increased, the corresponding portion was recovered. This was used as a column 1 eluted fraction.

### (3) ion exchange chromatography (column 2)

Then, the column 1 eluted fraction was passed through column 2 equilibrated with 50 mM Tris-HCl buffer (pH 7.8) containing 150 mM sodium chloride to allow adsorption. As column 2, Q-SepharoseFF (manufactured by GE Amersham Biosciences K.K.), which is a carrier for ion exchange chromatography, packed in a BPG200/500 empty column (manufactured by GE Amersham Biosciences K.K.) to diameter 20 cm, height 6.5 cm (2 L), was used. The linear flow rate was 70 cm/h. Furthermore, a 5-fold column volume of the equilibrating buffer was passed. For elution of HVJ from column 2, 50 mM Tris-HCl buffer (pH 7.8) containing 0.45 M sodium chloride was passed. At this time, since the absorbance of the column passing solution at UV 280 nm increased, the corresponding portion was recovered. This was used as a column 2 eluted fraction. Finally, 2 M sodium chloride was passed to regenerate column 2.

### (4) gel filtration chromatography (column 3)

The column 2 eluted fraction was passed through column 3 equilibrated with 50 mM Tris-HCl buffer (pH 7.8) containing 150 mM sodium chloride. As column 3, Sepharose6FF (manufactured by GE Amersham Biosciences K.K.), which is a carrier for gel filtration chromatography, packed in a BPG100/500 empty column (manufactured by GE Amersham Biosciences K.K.) to diameter 10 cm, height 33 cm (2.5 L), was used. The linear flow rate was 150 cm/h. Following the column eluted fraction, the equilibrating buffer was passed. At this time, since the absorbance of the column passing solution at UV 280 nm increased, the corresponding portion was recovered. This was used as a column 3 eluted fraction.

0.5% Methylcellulose in an amount corresponding to 1/4 volume was added to the column 3 eluted fraction to give an HVJ.solution containing a final concentration of 0.1% methylcellulose. This was passed through a 0.45 micron sterile filtration filter.

Of the main reagents used for buffer etc., tris(hydroxymethyl)aminomethane, sodium chloride, calcium chloride 2-hydrate, magnesium chloride 6-hydrate and sodium hydroxide used were special grade products manufactured by Wako Pure Chemical Industries, Ltd. or Sigma Ltd. Hydrochloric acid used was 6N standard product manufactured by Wako Pure Chemical Industries, Ltd. 0.5% Methylcellulose added as a stabilizer for a purified preparation was one manufactured by Wako Pure Chemical Industries, Ltd. For sterilization by filtration of a purified preparation, hydrophilic filtration filter Millipak Gamma Gold (Nihon Millipore K.K.) having a pore diameter of 0.45 micron was used.

### Example 2. activity measurement and recovery rate of purified HVJ

The activity of HVJ was measured based on the sialic acid degrading enzyme (neuraminidase, NA) activity and chicken red cell agglutinating activity (hemagglutinin activity, HA). Both are in a proportional relation with the concentration and amount of the recovered virus, and are quantitative. The residual activity of HVJ in Example 1 of the present invention and the recovery rate based on the NA activity value, as determined by these two methods, are shown in Table 1. The recovery rate using Comparative Example is shown in Table 2. From the comparison of Tables 1 and 2, the superior effect of the present invention is clear.

**(Table 1) (present invention, Example 1)**

| step | volume (mL) | NA activity (mU/mL) | total NA (mU) | recovery rate (NA %) | HA (U) |
|---|---|---|---|---|---|
| starting material | 10250 | 297.0 | 3044066 | 100.0 | |
| column 1 | 1200 | 1891.4 | 2269735 | 74.6 | |
| column 2 | 450 | 3524.0 | 1585790 | 52.1 | |
| column 3 | 500 | 3627.6 | 1813813 | 59.6 | |
| filtration | 625 | 3027.2 | 1892022 | 62.2 | |
| final product (inactivated virus) | 590 | 2066.1 | 1218993 | 40.0 | 5120 |

**(Table 2) (conventional purification method, Comparative Example 1)**

| step | volume (mL) | NA activity (mU/mL) | total NA (mU) | recovery rate (NA %) | HA (U) |
|---|---|---|---|---|---|
| starting material | 10800 | 71.9 | 775982 | 100.0 | |
| filtration | 10800 | 51.1 | 551397 | 71.1 | |
| membrane concentration | 600 | 903.4 | 542069 | 69.9 | |
| column 1 | 1000 | 573.4 | 573351 | 73.9 | |
| column 2 | 1000 | 373.8 | 373770 | 48.2 | |
| column 3 | 1050 | 393.8 | 413483 | 53.3 | |
| filtration | 1300 | 218.3 | 283804 | 36.6 | |
| final product (inactivated virus) | 1120 | 159.7 | 178864 | 23.1 | 2560 |

### Example 3. analysis of purity of purified HVJ by SDS-PAGE

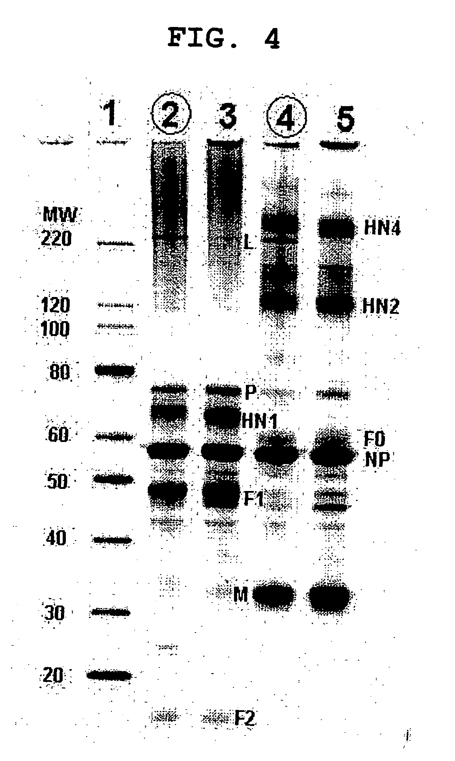

Electrophoresis of HVJ was performed on a 4-12% SDS-PAGE gel. HVJ was solubilized with a solubilizer (Sigma Ltd. CelLytic-M Cat. No. C2978) which is used for a membrane protein suitable for virus and directly electrophoresed. This was used as a nonreduction treatment sample. HVJ added with 2-mercaptoethanol after solubilization and heat treated at 95°C for 10 min was used as a reduction treatment sample. For comparison with general method, HVJ obtained by culture in a fertilized hen egg and purification of chorioallantoic fluid by anion exchange chromatography was treated in the same manner. The sample was electrophoresed at a constant voltage of 100V for 2.5 hr, stained with SYPRO Ruby fluorescence stain (Bio-Rad, Richmond, CA, SYPRO Ruby Protein Gel Stain Cat. No. 170-3125) and the gel was analyzed by a fluorescence imaging system. The results of the electrophoresis are shown in Fig. 4. As compared to the conventional method based on purification from hen egg, an HVJ envelope having the same purity could be obtained.

### Industrial Applicability

A method for purifying a virus envelope at a high recovery rate while maintaining its cell fusion activity can be provided. The purified virus envelope can be used as a vector for introducing a biopolymer such as gene and the like into a cell or a living organism.

According to the method of the present invention, an inactivated virus envelope (e.g., HVJ) can be purified at a high recovery rate as compared to conventional methods while maintaining its fusion activity. Therefore, it is useful for industrial production of a virus envelope. In addition, the method of the present invention can be applied to the purification of a non-inactivated envelope virus.

This application is based on a patent application No. 2004-219381 (filing date: July 27, 2004) filed in Japan, the contents of which are incorporated in full herein by this reference.

## Claims

1. A method of purifying a virus envelope, comprising use of hydrophobic chromatography.

2. The method of claim 1, wherein a functional group of said hydrophobic chromatography is a weak hydrophobic group.

3. The method of claim 1 or 2, wherein a functional group of said hydrophobic chromatography is an oligoethylene glycol group or a phenyl group.

4. The method of claims 1 to 3, wherein a functional group of said hydrophobic chromatography is an oligoethylene glycol group.

5. The method of claims 1 to 4, wherein said hydrophobic chromatography and ion exchange resin chromatography and/or gel filtration chromatography are combined.

6. The method of claim 5, wherein the ion exchange chromatography and hydrophobic chromatography are combined.

7. The method of claim 5 or 6, wherein an ion exchange resin is used for the first chromatography and a hydrophobic resin is used for the second chromatography.

8. The method of claims 5 to 7, wherein the ion exchange chromatography is anion exchange chromatography.

9. The method of claims 5 to 8, wherein the ion exchange chromatography is weak anion exchange chromatography.

10. The method of claims 5 to 9, wherein a functional group of the ion exchange chromatography is a diethylaminopropyl (DEAP) group.

11. The method of claims 5 to 10, wherein a functional group of the ion exchange chromatography is a low density substituted (Low Sub).

12. The method of claims 1 to 11, wherein a buffer for the chromatography has pH 7-9.

13. The method of claims 1 to 12, wherein a buffer for the chromatography has pH 7.8-8.5.

14. The method of claims 5 to 13, wherein a buffer for the ion exchange chromatography comprises sodium chloride or potassium chloride.

15. The method of claims 5 to 14, wherein a buffer for sample adsorption in the ion exchange chromatography comprises 0.01-150 mM of sodium chloride.

16. The method of claims 5 to 15, wherein a buffer for sample adsorption in the ion exchange chromatography comprises 30-70 mM of sodium chloride.

17. The method of claims 5 to 16, wherein a buffer for washing in the ion exchange chromatography comprises 150 mM-250 mM of sodium chloride.

18. The method of claims 5 to 17, wherein a buffer for washing in the ion exchange chromatography comprises 190-230 mM of sodium chloride.

19. The method of claims 5 to 18, wherein a buffer for elution in the ion exchange chromatography comprises 100 mM-1000 mM of sodium chloride.

20. The method of claims 5 to 19, wherein a buffer for elution in the ion exchange chromatography comprises 290-330 mM of sodium chloride.

21. The method of claims 1 to 20, wherein a buffer for the hydrophobic chromatography comprises ammonium sulfate, sodium sulfate or sodium chloride.

22. The method of claims 1 to 21, wherein a buffer for adsorption in the hydrophobic chromatography comprises 1.0 M-2.5 M of ammonium sulfate.

23. The method of claims 1 to 22, wherein a buffer for adsorption in the hydrophobic chromatography comprises 1.8-2.2 M of ammonium sulfate.

24. The method of claims 1 to 23, wherein a buffer for washing in the hydrophobic chromatography comprises 1.0-1.6 M of ammonium sulfate.

25. The method of claims 1 to 24, wherein a buffer for washing in the hydrophobic chromatography comprises 1.2-1.5 M of ammonium sulfate.

26. The method of claims 1 to 25, wherein a buffer for elution in the hydrophobic chromatography comprises 0.01-1.5 M of ammonium sulfate.

27. The method of claims 1 to 26, wherein a buffer for elution in the hydrophobic chromatography comprises 0.6-1.0 M of ammonium sulfate.

28. The method of claims 1 to 27, wherein a buffer for elution in the hydrophobic chromatography comprises a hydrophilic organic solvent.

29. The method of claim 28, wherein the hydrophilic organic solvent is polyvalent alcohol or lower alcohol.

30. The method of claim 28 or 29, wherein the polyvalent alcohol is ethylene glycol.

31. The method of claims 28 to 30, wherein a concentration of ethylene glycol is 0.01-50%.

32. The method of claims 28 to 31, wherein the concentration of ethylene glycol is 2-10%.

33. The method of claims 28 to 32, wherein the concentration of ethylene glycol is 3-7%.

34. The method of claims 1 to 33, wherein a buffer for elution in the hydrophobic chromatography comprises a surfactant.

35. The method of claims 1 to 34, wherein said surfactant is Tween 80 or Triton X.

36. The method of claims 1 to 35, wherein a buffer for elution in the hydrophobic chromatography comprises 0.001-1% of Tween 80.

37. The method of claims 1 to 36, wherein a buffer for elution in the hydrophobic chromatography comprises 0.01-0.1% of Tween 80.

38. The method of claims 1 to 37, wherein, in the hydrophobic chromatography, elution after sample adsorption is caused by lowering the temperature by not less than 5°C from the temperature during adsorption.

39. The method of claim 38, wherein the temperature during elution is within the range of (room temperature - 5)°C - 4°C.

40. The method of claims 1 to 39, wherein, in the hydrophobic chromatography, elution after sample adsorption is caused by raising the pH of the buffer for adsorption by not less than 0.5.

41. The method of claim 40, wherein the pH during elution is within the range of 6-10.

42. The method of claims 1 to 41, wherein gel filtration chromatography is conducted after the hydrophobic chromatography.

43. The method of claims 1 to 42, wherein a carrier of the gel filtration chromatography is Sepharose.

44. The method of claims 1 to 43, which comprises adding a divalent metal ion to a buffer used for the gel filtration chromatography.

45. The method of claim 44, wherein the divalent metal ion is calcium or magnesium.

46. The method of claim 44 or 45, wherein a concentration of the divalent metal ion is 0.1-10 mM.

47. The method of claims 44 to 46, wherein a concentration of the divalent metal ion is 1-2 mM.

48. The method of claims 1 to 47, wherein the virus belongs to a family selected from the group consisting of *Filoviridae, Bunyaviridae, Herpesviridae, Poxviridae, Togaviridae, Coronaviridae, Flaviviridae, Paramyxoviridae, Arenaviridae, Orthomyxoviridae, Retroviridae, Hepadnaviridae, Reoviridae* and *Deltaviridae.*

49. The method of claims 1 to 48, wherein the virus is selected from the group consisting of Ebola hemorrhagic fever virus, Crimean-Congo hemorrhagic fever virus, hantavirus, herpes simplex virus, EB virus, smallpox virus, cowpox virus, rubella virus, SARS virus (human coronavirus), hepatitis C virus, Japanese encephalitis virus, yellow fever virus, dengue fever virus, West Nile virus, Russian spring-summer encephalitis virus, hog cholera virus, rabies virus, vesicular stomatitis virus, Hemagglutinating Virus of Japan (HVJ), measles virus, epidemic parotiditis virus, mumps virus, rubella virus, RS virus, Lassa virus, influenza virus, human immunodeficiency virus (HIV), human T-cell leukemia virus type 1 (HTLV-1), feline immunodeficiency virus (FIV), hepatitis B virus (HBV), reovirus and hepatitis D virus.

50. The method of claims 1 to 49, wherein the virus is HVJ.

51. The method of claims 1 to 50, wherein the virus envelope is an attenuated or inactivated virus envelope.

52. The method of claims 1 to 51, wherein the virus envelope is an inactivated virus envelope.
